# EUROPEAN PATENT APPLICATION

(11) **EP 3 042 904 A1**
(43) Date of publication of application: **13.07.2016**
(21) Application number: 14841740.5
(22) Date of filing: 04.09.2014
(51) Int. Cl.: C07D 487/04, A61K 31/519, A61P 35/00

(54) **METHOD FOR PREPARING IMPROVED INTERMEDIATE FOR PRODUCING HIGH-PURITY PEMETREXED AND METHOD FOR PRODUCING HIGH-PURITY PEMETREXED USING INTERMEDIATE**

(30) Priority: 05.09.2013 KR 20130106497
(71) Applicant: Samyang Biopharmaceuticals Corporation, Seoul 110-725 (KR)
(72) Inventor: KIM, Young Min, Daejeon 305-762 (KR); KIM, Moon Suk, Daejeon 305-759 (KR); KIM, Seong Ho, Daejeon 300-825 (KR); CHO, Jin Suk, Daejeon 306-778 (KR)
(74) Representative: Wohlfahrt, Jan Günther
(86) International application number: PCT/KR2014/008331
(87) International publication number: WO 2015/034293

(57) **Abstract**

The present invention relates to a method for preparing an improved intermediate for producing high-purity pemetrexed and a method for producing high-purity pemetrexed using the intermediate, and more specifically, to a novel method for preparing pemetrexed diethyl ester, which is an intermediate for producing pemetrexed, or a salt thereof with high purity, and to a method for producing pemetrexed disodium salt with high purity using the intermediate.

## Description

### [TECHNICAL FIELD]

The present disclosure relates to an improved method for preparing an intermediate for preparing pemetrexed with high purity and a method for preparing pemetrexed with high purity by using the intermediate. More specifically, the present disclosure relates to a method for preparing pemetrexed diethyl ester or a salt thereof-which is an intermediate for preparing pemetrexed-with high purity and a method for preparing pemetrexed disodium salt with high purity by using the intermediate.

### [BACKGROUND ART]

Pemetrexed is N-(4-[2-(2-amino-4,7-dihydro-4-oxo-1H-pyrrolo[2,3-d]pyrimidin-5-yl)ethyl]benzoyl)-L-glutamic acid, and its disodium salt has the structure of the following formula 1.

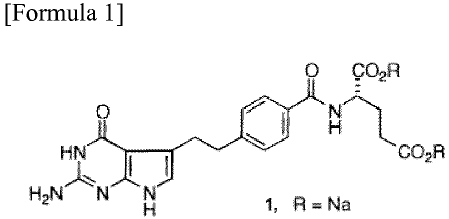

Pemetrexed shows antifolate activity and has been marketed by Eli Lilly and Company as an agent for treating lung cancer and pleural mesothelioma with the commercial name of Alimta™ (effective ingredient: pemetrexed disodium salt heptahydrate, aseptic freeze-dried powder for intravenous injection).

Pemetrexed disodium salt can be prepared according to the following reaction scheme 1 as disclosed in Korean Patent No. 0162654, and its preparation method is specifically disclosed in C.J. Barnett, et al., "A Practical Synthesis of Multitargeted Antifolate LY231514," Organic Process Research & Development, 3(3): 184-188 (1999).

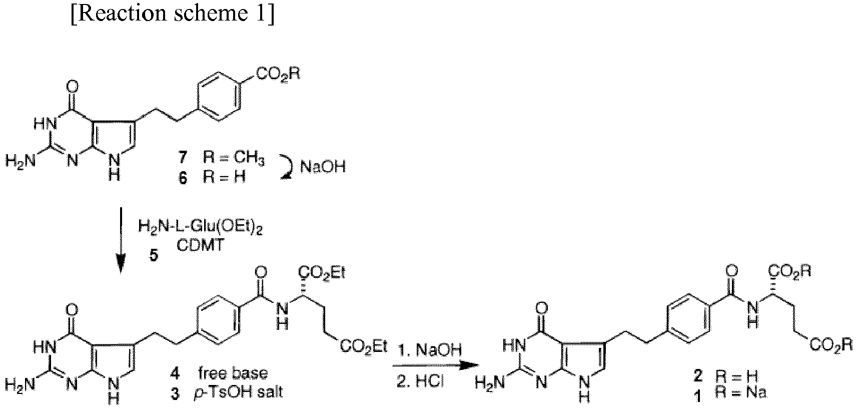

According to the above reaction scheme 1, pemetrexed disodium salt is prepared through the following steps:
1) The compound of formula 7 is hydrolyzed with NaOH aqueous solution, and the pH is then adjusted to about 4.4 with HCl aqueous solution to obtain the compound of formula 6 as a solid, and it is dried under vacuum.
2) To the compound of formula 6, dimethylformamide (DMF), N-methylmorpholine (NMM), 2-chloro-4,6-dimethoxy-1,3,5-triazine (CDMT) and L-glutamic acid diethyl ester HCl (LGA) are added and reacted at 25°C, the reaction product is then extracted and concentrated, and to the concentrated liquid, ethanol (EtOH) and p-toluenesulfonic acid monohydrate (PTSA) are added to obtain the intermediate compound of formula 3 as a solid of PTSA salt, which is then recrystallized under DMSO (dimethyl sulfoxide)/EtOH condition for further purification.
3) The compound of formula 3 is hydrolyzed with NaOH aqueous solution and the pH is then adjusted to about 3 with HCl aqueous solution to obtain the compound of formula 2-which is pemetrexed in free diacid form-as a solid, and it is dried under vacuum.
4) The compound of formula 2 is dissolved in NaOH aqueous solution (2-3 equivalents (eq.)) and HCl aqueous solution is added thereto, and EtOH is then added to obtain pemetrexed disodium salt (formula 1)-which is the desired product-as a solid.

The problem of the preparation method of pemetrexed disodium salt according to reaction scheme 1 is that heat generation occurs drastically when L-glutamic acid diethyl ester HCl is added at room temperature in order to form the intermediate pemetrexed diethyl ester (compound of formula 4). In addition, there is a problem of generating a large amount of byproduct in the room temperature reaction with the lapse of time.

Because of the above-stated problems, the pemetrexed diethyl ester (compound of formula 4) prepared according to the method of reaction scheme 1 is of low purity, having HPLC purity of 90% or lower, and even if it is prepared as PTSA salt (compound of formula 3) for purification, the HPLC purity is just at the level of 95% or lower. Furthermore, in HPLC purity analysis, the maximum amount of byproduct occurring at the relative retention time (RRT) of about 1.01-1.03 is 5-6%, and even after purification, the byproduct remains in an amount of 0.5% or more.

Such problems of low purity and byproduct generation can be improved by setting the reaction temperature lower than room temperature. For example, in preparation of the intermediate, if the reaction temperature is lowered to 0-15°C, through further purification the HPLC purity of pemetrexed diethyl ester can be raised to 97-98%, and the amount of byproduct at RRT of about 1.01-1.03 in HPLC purity analysis can be reduced to a level of 0.5% or less. However, even though the reaction temperature is lowered to 0-15°C and further purification is conducted, it is difficult to reduce the amount of byproduct at RRT of about 1.01-1.03 in HPLC purity analysis to a level of 0.15% or less. If the amount of this byproduct cannot be reduced to 0.15% or less during the preparation process of pemetrexed diethyl ester or a salt thereof, there may be a serious problem in which individual impurity remains in an amount of 0.1% or more in the final product, pemetrexed disodium salt. To prevent this, further purification of pemetrexed diacid is necessarily required.

In summary, the method for preparing pemetrexed disodium salt according to the above reaction scheme 1 has serious problems of low purity and byproduct generation in the intermediate preparation, and such problems are not improved to an acceptable level even by applying modifications such as lowering of reaction temperature and further purification. Therefore, in order to prepare pemetrexed disodium salt with high purity, a method for preparing the intermediate-which is different from the method of reaction scheme 1-is required.

### [CONTENTS OF THE INVENTION]

### [PROBLEMS TO BE SOLVED]

To resolve the above-stated problems of the prior arts, the present disclosure is , to provide a method for preparing pemetrexed diethyl ester or a salt thereof-which is an intermediate for preparing pemetrexed-with high purity, and a method for preparing pemetrexed disodium salt-which is the final product-with high purity and high yield by using the intermediate.

### [TECHNICAL MEANS]

To achieve the above-stated object, an embodiment provides a method for preparing pemetrexed diethyl ester of the following formula 4: 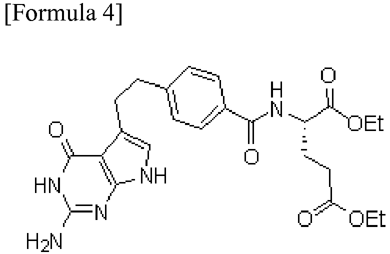 or a salt thereof, comprising:
(1) desalting L-glutamic acid diethyl ester hydrochloride salt of the following formula 5: 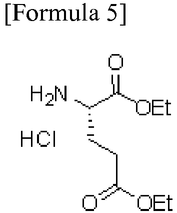 with aqueous solution of base, and extracting the desalted product by using organic solvent; and
(2) to the desalted L-glutamic acid diethyl ester obtained in step (1), adding dimethylformamide, compound of the following formula 6:

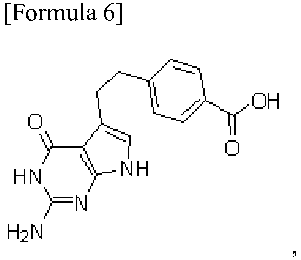

N-methylmorpholine and 2-chloro-4,6-dimethoxy-1,3,5-triazine in sequence, and reacting them.

A preferable embodiment provides the method for preparing pemetrexed diethyl ester or a salt thereof, further comprising: adding water and organic solvent to the resulting mixture of step (2), extracting the organic layer, and adding thereto ethanol and acid to obtain the final product as an acid salt of pemetrexed diethyl ester.

Another embodiment provides a method for preparing pemetrexed disodium salt of the following formula 1: 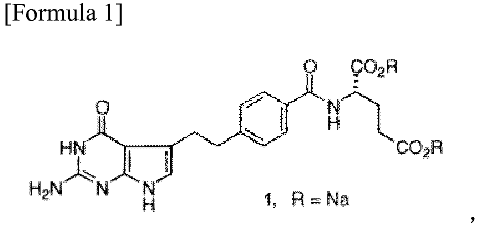 comprising:
(a) reacting pemetrexed diethyl ester or a salt thereof prepared by the above method with sodium hydroxide;
(b) adding acid to the product of step (a) to prepare pemetrexed diacid of the following formula 2: 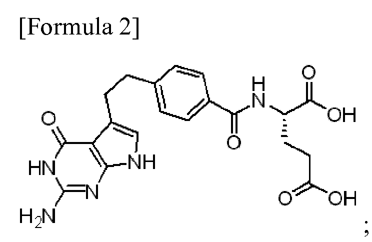 and
(c) reacting pemetrexed diacid prepared in step (b) with sodium hydroxide.

### [EFFECT OF THE INVENTION]

According to the present disclosure, pemetrexed diethyl ester or a salt thereof-which is useful as an intermediate for preparing pemetrexed disodium salt-can be prepared with high purity, and if the highly pure pemetrexed diethyl ester or a salt thereof prepared according to the present disclosure is used, highly pure pemetrexed diacid and highly pure pemetrexed disodium salt can be prepared with high efficiency even without further purification procedure, which is industrially very useful.

### [CONCRETE MODE FOR CARRYING OUT THE INVENTION]

Hereinafter, embodiments of the present disclosure will be described more specifically.

In the method for preparing pemetrexed diethyl ester (formula 4) or a salt thereof according to the present disclosure, L-glutamic acid diethyl ester hydrochloride salt (formula 5) is desalted with aqueous solution of base, and then the desalted L-glutamic acid diethyl ester is extracted by using organic solvent [step (1)].

As the aqueous solution of base used in desalting the L-glutamic acid diethyl ester hydrochloride salt, an aqueous solution of one or more bases selected from sodium hydrogen carbonate, potassium hydrogen carbonate, potassium carbonate, ammonia, N-methylmorpholine, calcium chloride and calcium carbonate can be preferably used, and more preferably, saturated aqueous solution of sodium hydrogen carbonate (NaHCO₃) can be used, but it is not limited thereto. Examples of the organic solvent used in extraction include a chlorinated solvent such as dichloromethane (DCM), carbon tetrachloride, chloroform, etc., an ether such as diethyl ether, methyl t-butyl ether(MTBE), etc., an aromatic solvent such as toluene, xylene, benzene, etc., an alcohol with 4 or more carbons such as butyl alcohol, pentyl alcohol, etc., a hydrocarbon with 4 or more carbons such as butane, pentane, hexane, heptane, etc., an ester such as ethyl acetate, or a combination of two or more of these solvents, but are not limited thereto. Desalting of L-glutamic acid diethyl ester hydrochloride salt can be conducted under conventional desalting conditions-for example, at room temperature for 10 minutes to 2 hours, but it is not limited thereto. After extracting the organic layer, without separate dehydration procedure, DMF solvent is further added and the mixture is concentrated under vacuum to remove 80% or more of the organic solvent for extraction (e.g., DCM), and the resulting product is used in the subsequent step.

To the desalted L-glutamic acid diethyl ester obtained in step (1), dimethylformamide (DMF), the compound of the above formula 6, N-methylmorpholine (NMM) and 2-chloro-4,6-dimethoxy-1,3,5-triazine (CDMT) are sequentially added in sequence, and reacted [step (2)].

The compound of the above formula 6 is preferably prepared from the compound of the following formula 7: 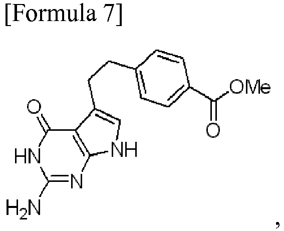 and accordingly, the method for preparing pemetrexed diethyl ester or a salt thereof of an embodiment of the present disclosure can further comprise preparing the compound of formula 6 from the compound of formula 7.

As for the equivalents (eq.) of the reagents used in the above step (2), based on 1 equivalent of the compound of formula 6, NMM is used preferably in 1.2-3.1 equivalents, more preferably in 1.5-3.0 equivalents, and most preferably in 2.9-3.0 equivalents; CDMT is used preferably in 1.0-1.4 equivalents, more preferably in 1.1-1.3 equivalents, and most preferably in 1.15-1.25 equivalents; and LGA is used preferably in 1.0-1.4 equivalents, more preferably in 1.1-1.3 equivalents, and most preferably in 1.15-1.25 equivalents.

In the above step (2), the temperature of adding the reagents can be 0-20°C, preferably 0-15°C, and more preferably 0-10°C. In addition, the reaction temperature after adding the reagents can be 0-27°C, preferably 0-20°C, more preferably 0-15°C, and still more preferably 5-15°C. The reaction time can be 5 minutes to 3 hours, preferably 10 minutes to 1 hour, and more preferably 30 minutes to 1 hour.

A preferable embodiment of the present disclosure further comprises: adding water (e.g., purified water) and organic solvent to the resulting mixture of the above step (2), extracting the organic layer, and adding ethanol (EtOH) and acid thereto to obtain the final product as an acid salt of pemetrexed diethyl ester.

As the solvent used in extracting the organic layer, dichloromethane (DCM) is preferable; and as the acid added to obtain the acid salt of pemetrexed diethyl ester, sulfuric acid, hydrochloric acid, tartaric acid or p-toluenesulfonic acid (PTSA) can be used, and preferably p-toluenesulfonic acid (PTSA) is used. In addition, the reaction of pemetrexed diethyl ester with acid is preferably conducted in a mixture solvent of dichloromethane (DCM), dimethylformamide (DMF) and ethanol (EtOH), and the reaction can be conducted at room temperature or higher (e.g., 40-60°C) for 30 minutes to 3 hours, for example.

The acid salt of pemetrexed diethyl ester prepared as such is then cooled, filtered, washed and dried under vacuum; thereby to obtain a salt of pemetrexed diethyl ester-for example, p-toluenesulfonic acid salt of pemetrexed diethyl ester represented by the following formula 3: 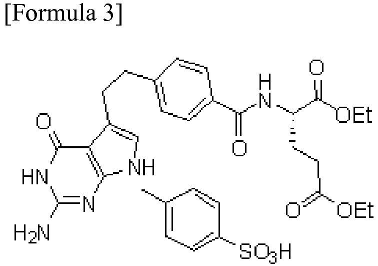 -with high purity of 99.0% or higher by HPLC.

According to the prior art, in order to prepare pemetrexed diethyl ester, the acid group of the compound of formula 6 was activated by using NMM and CDMT, and the compound of formula 5 was added thereto for amide coupling [Organic Process Research & Development 2005, 9, 738-742/Tetrahedron Lett. 1985, 26, 2901-2904]. However, as explained above, such a method had problems of lowering the purity of the desired product and generating byproduct due to the heat generated during the addition of the compound of formula 5. To complement this, the compound of formula 5 was added after desalting, but the problems of low purity of the desired product and byproduct generation were still unsolved thereby.

However, according to the present disclosure, if the compound of formula 5 is desalted and then the compound of formula 6 is added thereto-that is, if the reagents are introduced in the order reversed to that of the conventional method and the reaction is conducted-the reaction rate becomes higher and the purity of the target product can be raised remarkably higher (HPLC purity: 99.0% or higher, and more preferably 99.5% or higher) as compared with the conventional method, and at the same time the generation of byproduct can be reduced remarkably (amount of individual impurity occurring at RRT of about 1.01-1.03 in HPLC purity analysis: 0.15% or less, and more preferably 0.1% or less).

Another embodiment of the present disclosure provides a method for preparing pemetrexed disodium salt of the above formula 1 comprising the steps of: (a) reacting pemetrexed diethyl ester or a salt thereof prepared by the above method with sodium hydroxide; (b) adding acid to the product of step (a) to prepare pemetrexed diacid of the above formula 2; and (c) reacting pemetrexed diacid prepared in step (b) with sodium hydroxide.

In the above step (a), sodium hydroxide is preferably provided in an aqueous solution form (e.g., 1N aqueous solution), and the reaction of pemetrexed diethyl ester or a salt thereof and sodium hydroxide can be conducted preferably at 5-20°C, and more preferably 5-15°C, for 30 minutes to 3 hours, and more preferably 1 hour to 2 hours, for example.

In the above step (b), as the acid used, hydrochloric acid is preferable, and the addition amount thereof is preferably an amount to adjust the pH of the resulting solution after the acid addition to 2.8-3.2, and the reaction temperature of step (b) is preferably 5-25°C, and more preferably 5-15°C. The pemetrexed diacid prepared in step (b) is subjected to filtration and used in the subsequent step.

In the above step (c), sodium hydroxide is preferably provided in an aqueous solution form (e.g., 1N aqueous solution), and the reaction of pemetrexed diacid and sodium hydroxide can be conducted preferably at 5-25°C, and more preferably 5-15°C, for 30 minutes to 3 hours, and more preferably 30 minutes to 1 hour, for example. After the reaction, acid (e.g., hydrochloric acid) can be added to adjust the pH to 7.5-8.5.

According to an embodiment of the present disclosure, for the pemetrexed disodium salt prepared in the above step (c), workup procedures-for example, crystallization (50-60°C in EtOH), cooling, filtration, washing, vacuum drying, etc.-can be conducted, and as a result, it is possible to obtain pemetrexed disodium salt with high purity of 99.9% or higher by HPLC and 0.05% or less of individual impurity content.

In summary, the method for preparing pemetrexed disodium salt of formula 1 provided by an embodiment of the present disclosure comprises: (i) desalting L-glutamic acid diethyl ester hydrochloride salt of the above formula 5 with aqueous solution of base, and extracting the desalted product by using organic solvent; (ii) to the desalted L-glutamic acid diethyl ester obtained in step (i), adding dimethylformamide, the compound of the above formula 6, N-methylmorpholine and 2-chloro-4,6-dimethoxy-1,3,5-triazine in sequence, and reacting them to prepare pemetrexed diethyl ester of the above formula 4 or a salt thereof; (iii) reacting pemetrexed diethyl ester or a salt thereof prepared in step (ii) with sodium hydroxide; (iv) adding acid to the product of step (iii) to prepare pemetrexed diacid of the above formula 2; and (v) reacting pemetrexed diacid prepared in step (iv) with sodium hydroxide.

Embodiments of the present disclosure are explained in more detail through the following Examples. However, the following Examples are intended only to illustrate the embodiments, and the scope of the present disclosure is not limited thereby.

### [EXAMPLES]

The HPLC (high performance liquid chromatography) methods of monitoring the purities of pemetrexed diethyl ester and pemetrexed diacid or disodium salt prepared in the following Examples, are as follows:

### (1) Method of monitoring the purity of pemetrexed dimethyl ester

a) HPLC: HP 1100 series with Chemstation
b) Flow rate: 1.0 mL/min
c) Injection volume: 10 mL
d) Detection: at 250 nm
e) Column temperature: 20°C
f) Column

| Manufacturer | YMC |
|---|---|
| Type | ODS-AC |
| Size | 4.6 mm x 15 cm |
| Stationary Phase | 3 µm packing |

g) Mobile phase
- Solution A: 0.2% trifluoroacetic acid (TFA) in 100% water
- Solution B: 0.2% trifluoroacetic acid (TFA) in 100% acetonitrile (ACN)

| Time (min) | Solution A (%) | Solution B (%) |
|---|---|---|
| 0 | 85 | 15 |
| 2 | 85 | 15 |
| 30 | 77 | 23 |
| 50 | 60 | 40 |
| 51 | 85 | 15 |
| 55 | 85 | 15 |

h) Test solution preparation
- Diluent preparation: Diluent was the mixed solution of Diluent A/B = 1/1.
- Diluent A: About 4.0 g of sodium phosphate dibasic anhydrous (Na₂HPO₄) was weighed and dissolved in 1 L of ultrapure water (pH: 8.5-9.5).
- Diluent B: Acetonitrile

### (2) Method of monitoring the purity of pemetrexed diacid or disodium salt

a) HPLC: HP 1100 series with Chemstation
b) Flow rate: 0.5 mL/min
c) Injection volume: 10 mL
d) Detection: at 250 nm
e) Column temperature: 20°C
f) Column

| Manufacturer | YMC |
|---|---|
| Type | ODS-AC |
| Size | 4.6 mm x 15 cm |
| Stationary Phase | 3 µm packing |

g) Mobile phase
- Solution A: 0.2% TFA in 100% water
- Solution B: 0.2% TFA in 100% ACN

| Time (min) | Solution A (%) | Solution B (%) |
|---|---|---|
| 0 | 85 | 15 |
| 4 | 85 | 15 |
| 22 | 80 | 20 |
| 38 | 50 | 50 |
| 38.5 | 85 | 15 |
| 45 | 85 | 15 |

h) Test solution preparation
- Diluent A: About 4.0 g of sodium phosphate dibasic anhydrous (Na₂HPO₄) was weighed and dissolved in 1 L of ultrapure water (pH: 8.5-9.5).
- Diluent B: Acetonitrile
- Diluent for pemetrexed diacid: Mixed solution of Diluent A/B = 1/1
- Diluent for pemetrexed disodium salt: Ultrapure water

### Example 1: Preparation of pemetrexed diethyl ester p-toluenesulfonic acid salt (formula 3)

In a reactor, 1 L of saturated aqueous solution of NaHCO₃ and 88 g of L-glutamic acid diethyl ester hydrochloride salt (LGA) were added and stirred at room temperature for 30 minutes, and then 1 L of DCM was added thereto and the organic layer was extracted. To the separated organic layer, 0.5 L of DMF was added, and the mixture was concentrated at 20-30°C to remove 80% or more of DCM. To the concentrated solution, 93 g of 4-[2-(2-amino-4,7-dihydro-4-oxo-1H-pyrrolo[2,3-d]pyrimidin-5-yl)ethyl]benzoic acid (formula 6, HPLC purity: 98.3%) was added and the mixture was cooled to 0-10°C, and then 93 g of N-methylmorpholine (NMM) and 65 g of 2-chloro-4,6-dimethoxy-1,3,5-triazine (CDMT) were sequentially added thereto. After stirring the reaction mixture at 5-15°C for 1 hour, 1 L of DCM and 1 L of purified water were added thereto and the organic layer was extracted (HPLC purity: 97.9%). To the extracted solution, 0.3 L of DMF, 3.3 L of EtOH and 148 g of p-toluenesulfonic acid were added, and the mixture was stirred at about 50°C for 2 hours. The formed solid was cooled to 30°C and filtered, washed with 1 L of EtOH, and dried under vacuum at 50-60°C for 16 hours to obtain 173 g of pemetrexed diethyl ester p-toluenesulfonic acid salt as white solid (Yield: 85%, HPLC purity: 99.5%, individual impurity content: < 0.1%).

### Comparative Example 1: Preparation of pemetrexed diethyl ester p-toluenesulfonic acid salt (formula 3)

According to the method of Organic Process Research & Development, 3(3): 184-188 (1999), pemetrexed diethyl ester p-toluenesulfonic acid salt was prepared by using 10 g of 4-[2-(2-amino-4,7-dihydro-4-oxo-1H-pyrrolo[2,3-d]pyrimidin-5-yl)ethyl]benzoic acid (formula 6, HPLC purity: 98.3%). The HPLC purity of the organic layer after the reaction was 85.4% (RRT 1.02: 5.7%), and the HPLC purity of the prepared p-toluenesulfonic acid salt was 93.6% (RRT 1.02: 3.3%).

### Example 2: Preparation of pemetrexed diacid (formula 2)

In a reactor, 1 L of 1N NaOH aqueous solution was added, and 143 g of the compound of formula 3 prepared in Example 1 was added thereto at 5-15°C. After stirring at 5-15°C for 2 hours, the mixture was filtered (HPLC purity: 99.8%). To the filtered solution, 2 L of EtOH was added, and 2N HCl aqueous solution was slowly added thereto dropwise at 5-15°C to adjust pH to 3.0. The formed crystalline mixture was stirred at 40-50°C for 1 hour and filtered at 40°C. The filtered product was washed with 2 L of purified water, and further washed with 1 L of EtOH. The filtered product as obtained was added to 4 L of EtOH/purified water (1:1, v/v), and the mixture was stirred at 40-50°C for 1 hour, and cooled and filtered at room temperature. The filtered product was washed with 2 L of purified water, further washed with 1 L of EtOH, and dried under vacuum at 40-45°C for 16 hours to obtain 88 g of pemetrexed diacid as white solid (Yield: 95%, HPLC purity: 99.9%, individual impurity content: < 0.05%).

### Example 3: Preparation of pemetrexed disodium salt (formula 1)

In a reactor, 1N NaOH aqueous solution was added, and 87 g of the compound of formula 2 prepared in Example 2 was added thereto at 5-15°C. After stirring for 30 minutes, the mixture was filtered. To the filtered solution, 0.5N HCl aqueous solution was slowly added dropwise to adjust the pH to 7.5-8.5. The solution was heated to 50-60°C and 7 L of EtOH was slowly added thereto dropwise to form crystals. The formed white solid was slowly cooled to room temperature and filtered, washed with 1 L of EtOH/purified water mixture solution (4:1, v/v), and dried under vacuum at 40°C for 21 hours to obtain 91 g of pemetrexed disodium salt as white solid (Yield: 87%, HPLC purity: > 99.9%, any other single impurity content: < 0.05%). From the water content of 9.05% by weight measured by the Karl Fischer method, the product was confirmed as 2.5 hydrate (requirement standard: 8.5-11% by weight).

## Claims

1. A method for preparing pemetrexed diethyl ester of the following formula 4: 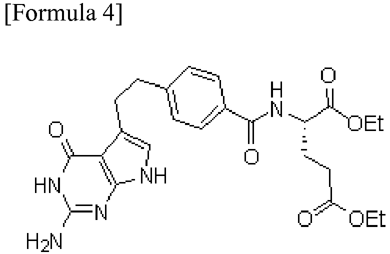 or a salt thereof, comprising:
(1) desalting L-glutamic acid diethyl ester hydrochloride salt of the following formula 5: 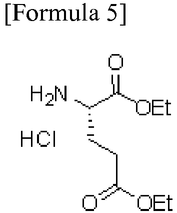 with aqueous solution of base, and extracting the desalted product by using organic solvent; and
(2) to the desalted L-glutamic acid diethyl ester obtained in step (1), adding dimethylformamide, compound of the following formula 6: 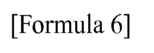 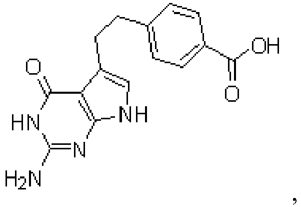 N-methylmorpholine and 2-chloro-4,6-dimethoxy-1,3,5-triazine in sequence, and reacting them.

2. The method of claim 1, wherein in step (1), the base is one or more selected from sodium hydrogen carbonate, potassium hydrogen carbonate, potassium carbonate, ammonia, N-methylmorpholine, calcium chloride and calcium carbonate, and the organic solvent is dichloromethane.

3. The method of claim 1, wherein the compound of formula 6 is prepared from the compound of the following formula 7:

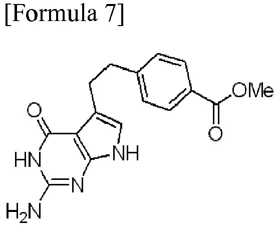

4. The method of claim 1, further comprising: adding water and organic solvent to the resulting mixture of step (2), extracting the organic layer, and adding thereto ethanol and acid to obtain the final product as an acid salt of pemetrexed diethyl ester.

5. The method of claim 4, wherein the acid is p-toluenesulfonic acid.

6. The method of claim 4, wherein the reaction of pemetrexed diethyl ester and acid is conducted in a mixed solvent of dichloromethane, dimethylformamide and ethanol.

7. The method of claim 4, wherein the obtained acid salt of pemetrexed diethyl ester has HPLC purity of 99.0% or higher.

8. A method for preparing pemetrexed disodium salt of the following formula 1: 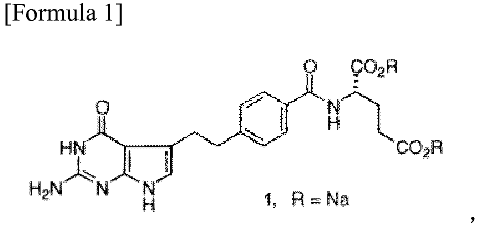 comprising:
(a) reacting pemetrexed diethyl ester or a salt thereof prepared by a method of any of claims 1 to 7 with sodium hydroxide;
(b) adding acid to the product of step (a) to prepare pemetrexed diacid of the following formula 2: 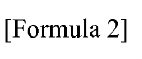 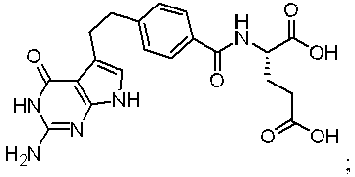 and
(c) reacting pemetrexed diacid prepared in step (b) with sodium hydroxide.

9. The method of claim 8, wherein in step (b), the pH of the resulting solution after adding acid is 2.8-3.2.

10. The method of claim 8, wherein after the reaction in step (c), acid is added thereto for adjusting the pH to 7.5-8.5.

11. The method of claim 8, wherein the obtained pemetrexed disodium salt has HPLC purity of 99.9% or higher and individual impurity content of 0.05% or less.
